# EUROPEAN PATENT SPECIFICATION

(11) **EP 4 481 056 B1**
(45) Date of publication and mention of the grant of the patent: **04.03.2026**
(21) Application number: 24210856.1
(22) Date of filing: 21.08.2020
(51) Int. Cl.: C12Q 1/6809

(54) **METHOD OF HAPLOTYPING**
HAPLOTYPISIERUNGSVERFAHREN
PROCÉDÉ D'HAPLOTYPAGE

(30) Priority: 22.08.2019 GB 201912103
(43) Date of publication of application: 25.12.2024
(62) Divisional of application: 20761882.8
(73) Proprietor: Oxford University Innovation Limited, Oxford, OX2 0JB (GB)
(72) Inventor: HUGHES, James R., Oxford OX3 9DS (GB)
(74) Representative: Mathys & Squire

(56) References cited:
- EP-A1- 1 829 979
- LEE JAMES C ET AL: "Human SNP Links Differential Outcomes in Inflammatory and Infectious Disease to a FOXO3-Regulated Pathway", CELL, ELSEVIER, AMSTERDAM, NL, vol. 155, no. 1, 12 September 2013 (2013-09-12), pages 57 - 69, XP028729732, ISSN: 0092-8674, DOI: 10.1016/J.CELL.2013.08.034
- S. SIGURDSSON ET AL: "A risk haplotype of STAT4 for systemic lupus erythematosus is over-expressed, correlates with anti-dsDNA and shows additive effects with two risk alleles of IRF5", HUMAN MOLECULAR GENETICS, vol. 17, no. 18, 1 January 2008 (2008-01-01), pages 2868 - 2876, XP055039064, ISSN: 0964-6906, DOI: 10.1093/hmg/ddn184
- BRANDON J THOMAS ET AL: "Allele-specific transcriptional elongation regulates monoallelic expression of the IGF2BP1 gene", EPIGENETICS & CHROMATIN, BIOMED CENTRAL LTD, LONDON, UK, vol. 4, no. 1, 3 August 2011 (2011-08-03), pages 14, XP021109674, ISSN: 1756-8935, DOI: 10.1186/1756-8935-4-14
- DAN?B. RAINBOW ET AL: "Commonality in the genetic control of Type?1 diabetes in humans and NOD mice: variants of genes in the IL-2 pathway are associated with autoimmune diabetes in both species", BIOCHEMICAL SOCIETY TRANSACTIONS, vol. 36, no. 3, 1 June 2008 (2008-06-01), pages 312, XP055084272, ISSN: 0300-5127, DOI: 10.1042/BST0360312

## Description

The present invention relates to a method of identifying mutations in alleles of a gene which may be causative of dysregulation of the expression levels of the alleles of the gene in a target eukaryotic cell.

The present disclosure also relates to detecting aberrant expression of genes which may be associated with a disease or disorder using haplotype phasing. In particular, the disclosure also relates to a method of obtaining an indication of dysregulation between the expression levels of at least two alleles of a gene in a target eukaryotic cell.

Common variant genome-wide association studies (GWAS) have identified tens-of-thousands of genetic variants associated with complex traits (e.g. body mass index, height, etc.) and predisposition to common diseases (e.g. diabetes, heart disease, immune disorders, and cancer predispositions). Despite statistically-robust genetics, identifying the causative variants and genes underlying GWAS signals has been frustrated by two broad factors. First, the causal sequences have been complex to pin down precisely due to the extensive linkage of sequence variation within the genome; and second, irrespective of the particular GWAS in question, the vast majority of variants lie outside of the interpretable coding genome.

The ability to interpret the non-coding genome is a priority for the biomedical sciences. In the last decade, our understanding of non-coding DNA has evolved from "junk" into the complex regulatory wiring that controls the expression and function of the coding and non-coding transcriptome. Central to this function are regulatory non-coding elements: promoters, enhancers and intron/exon boundaries. Although the existence of some regulatory elements has been known for several decades, their general ubiquity - with key tissue-specific roles in development and routine cellular processes - has only become clear in the last two decades. The large overlap between these elements and the genetics of common disease has started to make sense of the confounding non-coding distribution of GWAS signals.

In particular, further studies of the transcriptome (i.e. the set of all RNA molecules in one cell or a population of cells) are needed in order to help to identify genes which are aberrantly expressed in specific diseases (or their associated biochemical pathways) and hence to identify candidate drug targets.

Aberrant expression of genes may be due to changes in the elements which regulate the expression of the genes. Changes in these elements may lead to the over- or under-expression of the genes, changes in the temporal expression of the genes or in their tissue specificity.

It is known that such changes may also lead to dysregulation in the levels of expression of different alleles of the same gene within a single cell or population of cells; and that such dysregulation may also be associated with specific diseases or disorders.

The determination of the linkage between sequence variation across the alleles within a cell (i.e. the haplotype) is known as "phasing"; and a finding of different expression levels of the alleles is known as "allelic skew".

Traditionally, the majority of biochemical and genetic analysis of mRNA is performed on polyA⁺ mRNA. Total RNA is first extracted from cells; it is then passed down an oligo-dT column to bind the polyA⁺ mRNA; and the polyA⁺ mRNA is then selectively eluted from the column. PolyA⁺ RNA contains the coding sequence of the mRNA, i.e. the (non-coding) introns have been removed, and this is the form of mRNA which is usually of most interest to the researcher. The use of a poly-dT column also has the advantage that rRNA (which is an unwanted contaminant and which is abundantly expressed in all cells) is removed by this method.

The inventors have now recognised that additional information from the transcriptome may be obtained by using mRNA which is not limited to polyA⁺ RNA. More specifically, they have recognised that, if pre-mRNA is used (e.g. polyA⁻ mRNA, which still contains introns), then the natural variations in genome sequences which are found in introns and down-stream sequences and which are removed in polyA⁺ RNA can be used to determine allelic skew and so identify dysregulated genes. Importantly, when performed in a phased genome sequence, all sequence variants can be attributed to a specific allele to determine allelic skew across the whole gene and link these with sequence variation outside the body of the gene.

Whilst the isolation of polyA⁻ mRNA is previously known (e.g. Kowalczyk, 2012), it has not previously been used in the context of gene haplotyping or allelic skew.

EP 1829979 A1 relates methods for searching for gene polymorphisms (e.g. SNPs) in cDNAs as a means to identify genes whose expression levels are different between alleles. The inventors recognised that mature poly A+ mRNA had only one exon sequence following splicing and hence such sequences were "too short" to comprise enough SNPs to be evaluated. The inventors therefore selected intra-nuclear RNA in order to provide a "long strand" which was expected to contain many genetic polymorphisms that might enable a gene whose expression level is different between alleles to be distinguished (see paragraphs [0006] and [0018]).

James *et al.* (2013) uses pre-RNA to detect skew in the expression of one allele relative to another under stimulated conditions in immune cells, but does not do so in the context of a phased genome to link with genetics or to test for functional skew in the epigenome within or distal to a gene.

Sigurdsson *et al.* (2008) used allelic skew in the context of an unphased genome to detect allelic skew with a risk haplotype that lies within the body of the gene, but does not do so in the context of a phased genome to link with genetics or to test for functional skew in the epigenome within or distal to a gene.

Thomas *et al.* (2011) combines the uses of epigenetic skew and gene expression skew to look at the epigenetic effects associated with a gene which is mono-allelic, but only within the body of the gene as they do not use this in the context of a phased genome.

Rainbow *et al.* (2008) uses a form of pre-mRNA to look at the allele-specific differences in expression of the IL-2 gene, but this is not in the context of a phased genome to link with the distal regulatory landscape or genetics.

The invention is set out in the appended set of claims. In particular, the invention provides a method of identifying mutations in alleles of a gene which may be causative of dysregulation of the expression levels of the alleles of the gene in a target eukaryotic cell.

The disclosure also relates to a method of obtaining an indication of dysregulation between the expression levels of different alleles of the same gene in a target eukaryotic cell, the method comprising the use of pre-mRNA. In a phased genome, this dysregulation can then be linked to sequence variation outside of the gene body which causes the gene dysregulation on the affected allele.

Without phasing, each SNP detected in the RNA analysis (unless they are close together, i.e. within a short distance <300bp) cannot be assigned to the same allele without the assumption that all SNPs are skewed in the same direction. Therefore they cannot be used to add statistical robustness to the allelic skew of a given allele. With phasing, all of the SNPs can be assigned to a given allele and can be seen statistically to behave similarly, in terms of changes in representation in the RNA and the direction of skew.

More importantly, without phasing, this skewed RNA expression cannot be linked to changes outside of the gene. The regulatory elements that control gene expression in *cis* on the same chromosome can be situated up to 2 million base pairs away from the gene they control. Using phasing, the genotypes that genetically link a sequence change with a given trait or diseases can be seen to be on the same allele as a gene that behaves reproducible in its allelic skew in expression. This also allows one to link epigenetic changes at the distal SNP with expression changes of the allele on the same chromosome. These epigenetic changes can be detected using allelic skew for sequence-based genomic assays that measure enhancer activity, such as open chromatin assays (e.g. DNAse-seq or ATAC-seq), chromatin marks associated with regulatory activity (e.g. ChIP-seq for chromatin marks such as H3k27ac, H3k4me1 or for protein binding such as RNA polymerase, transcription factor binding) or the binding of important regulatory structural proteins, such as CTCF.

The present disclosure relates to detecting aberrant expression of genes which may be associated with a disease or disorder using haplotype phasing.

In some embodiments, the method of the invention further comprises detecting aberrant expression of genes which may be associated with a disease or disorder using haplotype phasing.

Using the method of the invention, it becomes possible to link the skew in gene expression to genetic signals associated with traits and disease traits outside the body of the gene, where they are generally enriched. It also allows for the linking of the skew in gene expression to skew in sequence-based genomics assays linked to regulatory activities, and can do so *en masse* and at genome-scale, to validate the mechanism underlying the changes in gene expression.

The information which is obtained from the methods of the invention may be used to identify genes which are aberrantly expressed in specific diseases or disorders (or their associated biochemical pathways) and hence to identify candidate drug targets.

This information may also be useful in order to help to determine the underlying genetic cause of the disease or disorder.

The disclosure relates to a method of obtaining an indication of dysregulation between the expression levels of at least two alleles of the same gene in a target eukaryotic cell, the method comprising the steps of:
for a plurality of genes from one or more target eukaryotic cells,
   (a) obtaining pre-mRNAs of at least two alleles of the same gene; and
   (b) determining the ratios (R_{i,j}) between amounts of the pre-mRNAs of one or more pairs of alleles (i,j) of the same gene;
wherein if R_{i,j} ≠ 1 for one or more pairs of alleles (i,j) of the same gene, then this is indicative of dysregulation between the expression levels of those two alleles of that gene in that target eukaryotic cell.

Preferably, if R_{i,j} is < 0.9 or if R_{i,j} > 1.1 for one or more pairs of alleles (i,j) of the same gene, then this is indicative of dysregulation between the expression levels of those two alleles of that gene in that target eukaryotic cell.

The disclosure relates to a method of identifying genes whose alleles are dysregulated in a target eukaryotic cell, the method comprising the steps of:
for a plurality of genes from one or more target eukaryotic cells,
   (a) obtaining pre-mRNAs of at least two alleles of the genes; and
   (b) determining the ratios (R_{i,j}) between the amounts of pre-mRNAs of pairs of alleles (i,j) of the genes;
wherein if R_{i,j} ≠ 1 for a pair of alleles (i,j) of a gene, then this is indicative of a gene whose alleles are dysregulated in the target eukaryotic cell.

Preferably, if R_{i,j} is < 0.9 or if R_{i,j} > 1.1 for a pair of alleles (i,j) of a gene, then this is indicative of a gene whose alleles are dysregulated in the target eukaryotic cell.

The disclosure relates to a method of obtaining an indication of the cause of a disease or disorder, the method comprising the steps of:
for a plurality of genes from one or more target eukaryotic cells,
   (a) obtaining pre-mRNAs of at least two alleles of the genes; and
   (b) determining the ratios (R_{i,j}) between the amounts of pre-mRNAs of pairs of alleles (i,j) of the genes;
wherein the target eukaryotic cells are ones which are associated with or characteristic of a disease or disorder,
and wherein if R_{i,j} ≠ 1 for a pair of alleles (i,j) of a gene, then this is indicative that the disease or disorder is caused by dysregulation between the expression levels of the alleles of that gene.

Preferably, if R_{i,j} is < 0.9 or if R_{i,j} > 1.1 for a pair of alleles (i,j) of a gene, then this is indicative that the disease or disorder is caused by dysregulation between the expression levels of the alleles of that gene.

The disclosure relates to a method of identifying mutations in alleles of a gene which may be causative of dysregulation of the expression levels of the alleles of the gene in a target eukaryotic cell, the method comprising the steps of:
for a plurality of genes from one or more target eukaryotic cells,
   (a) obtaining pre-mRNAs of at least two alleles of the genes; and
   (b) determining the ratios (R_{i,j}) between the amounts of pre-mRNAs of one or more pairs of alleles (i,j) of the genes;
wherein when R_{i,j} ≠ 1 for a pair of alleles (i,j) of a gene, or in response to determining that R_{i,j} ≠ 1 for a pair of alleles (i,j) of a gene, the method additionally comprises the steps:
   (c) determining the nucleotide sequences of that pair of alleles; and
   (d) comparing the nucleotide sequences of that pair of alleles in order to identify differences between the nucleotide sequences of that pair of alleles;
wherein one or more of the differences between the nucleotide sequences of the pair of alleles of the gene may be mutations which are causative of the dysregulation of the expression levels of the two alleles of that gene in the target eukaryotic cell.

Preferably, when R_{i,j} is < 0.9 or if R_{i,j} > 1.1 for a pair of alleles (i,j) of a gene, or in response to determining that R_{i,j} is < 0.9 or if R_{i,j} > 1.1 for a pair of alleles (i,j) of a gene, the method comprises the steps:
(c) determining the nucleotide sequences of that pair of alleles; and
(d) comparing the nucleotide sequences of that pair of alleles in order to identify differences between the nucleotide sequences of that pair of alleles;
wherein one or more of the differences between the nucleotide sequences of the pair of alleles of the gene may be mutations which are causative of the dysregulation of the expression levels of the two alleles of that gene in the target eukaryotic cell.

As used herein, the term "dysregulation" refers to differences in regulation between one or more alleles of a gene. Ordinarily, alleles of a gene are expressed at similar levels. However, mutations in the regulatory regions which control the expression of the individual alleles may result in enhanced or reduced expression of those alleles. The term "dysregulation" therefor also refers to differences in the expression levels of alleles of a gene.

The target cells are eukaryotic cells (i.e. cells whose nuclear DNA has introns). Preferably, the eukaryotic cells are mammalian cells, e.g. human, monkey, mouse, rat, pig, goat, horse, sheep or cow. Most preferably, the cells are human cells.

The cells may be cells primary cells or cells from a cell line. Preferably, the cells are primary cells.

In some embodiments of the invention, the target cells are haematopoietic cells, e.g. erythrocytes, lymphocytes (e.g. T-cells, B-cells and natural killer cells), granulocytes, megakaryocytes and macrophages. Preferably, the target cells are primary lymphoid cells.

In other embodiments, the target cells are brain cells; preferably the target cells are primary neuronal cells.

Whilst the cells are preferably diploid cells, the methods of the invention are applicable to cells of other ploidies, e.g. tetraploid cells.

In some embodiments of the invention, the target eukaryotic cells are ones which are associated with or characteristic of a disease or disorder. Examples of eukaryotic cells which are associated with or characteristic of a disease or disorder are given below:

| **Eukaryotic cells** | **Disease or disorder** |
|---|---|
| cancer cells | Cancer |
| Neuronal cells | Dementia, Alzheimer's disease and psychiatric disorders (e.g. schizophrenia) and autistic spectrum disorders |
| Pancreatic cells | Diabetes |
| Immune cells | Autoimmune disease and infection |
| Erythroid cells | Malaria |
| Cardiac muscle cells | Cardiac arrhythmias, cardiomyopathies |
| Pulmonary cells | Lung disease, including asthma |
| Skin cells | Dermatological conditions, such as psoriasis and eczema |
| Adipocytes | Obesity |
| Retina | Retinal degeneration |
| Cells from the upper and lower gastrointestinal tract | Inflammatory bowel disease, irritable bowel syndrome |
| Hepatic cells | Diabetes, non-alcoholic steatohepatitis, liver diseases |
| Endothelial and other vascular cell types | Cardiovascular disease, including stroke and ischaemic heart disease |

Examples of diseases and disorders include those in the above table.

The pre-mRNA is obtained from one or more target eukaryotic cells. In some preferred embodiments, the pre-RNA is obtained from single target cells. In other embodiments, the RNA is obtained from a population of target cells. In such embodiments, population of target cells comprises substantially or entirely the same type of cells, i.e. the population is substantially or entirely homogenous.

The genes are ones which are present in the target eukaryotic cell. As used herein, the term "gene" is not limited to the RNA-coding or protein-coding sequence. It includes associated regulatory elements, e.g. enhancers, promoters and terminator sequences. The term "gene" may be defined as including its transcribed regions, its intron, promoter regions and all regulatory or structural elements that determine its activity or level of expression within 2 million base pairs of the promoters of the transcribed portion of the gene.

The methods of the invention are particularly applicable to genes which are affected by genetic variation that is known to be associated with a disease or trait. The genetic variation will generally have been identified by genome-wide association studies (GWAS), but the genetic variation could also be mutation in an individual subject or in a cancerous subclone.

Although the invention is capable of detecting dysregulation in any gene, certain classes of dysregulated genes are preferred. These include genes that are expressed specifically in the cell-type of interest compared to genes expressed in all cell types. Similarly genes that are important for the function of a cell type of interest, for example genes of the immune synapse in immune cells, are also preferred. Also preferred are genes that encode transcription factors that determine the cell-type identity and/or behaviour of a given cell type. Additionally, genes that affect the overall fitness of the cell such as cell-cycle regulating genes or gene related to apoptosis are preferred. In cancer cells, genes that affect processes such as cell proliferation, mobility and/or adhesion including but not limited to membrane receptors, secondary messenger molecules, transcription factors and epigenetic regulators are preferred.

As used herein, the term "plurality of genes" refers to 2 or more genes, e.g. 2-10, 10-100, 100-500, 500-1000, 1000-5000, 5000-10000, or 10000 or more.

There are at least two alleles of the same gene in each target eukaryotic cell. For example, there may be 2, 3 or 4 alleles of the same gene. Preferably, there are 2 alleles of the same gene in each target eukaryotic cell.

The sequences of one or more or all of the exons in the pre-mRNA alleles may be the same or different. The sequences of one or more of all of the introns in the pre-mRNA alleles may be the same or different.

The target cells are preferably heterozygous for one or more of the alleles of interest.

Step (a) of the method of the invention comprises the step of obtaining pre-mRNA. Preferably, the pre-mRNAs are from at least two alleles of the same gene on a given haplotype.

Pre-mRNA is the first form of RNA which is created through transcription in the process of protein synthesis. Pre-mRNA is transcribed from a DNA template in the target cell nucleus. The pre-RNA will generally be 5'-capped (i.e. with a 7-methylguanosine) because this capping occurs within a few nucleotides after the initiation of RNA synthesis. In the context of this invention, the pre-mRNA may or may not comprise a 5'-cap.

There are two main differences between pre-mRNA and mRNA in eukaryotic cells: (i) a poly-A tail is added to the 3' end of the pre-mRNA; and (ii) introns are spliced out of the pre-mRNA. Once the pre-mRNA has been processed to include the above features, it is termed "mature messenger RNA" or simply "messenger RNA" (mRNA). Polyadenylated mRNA is also known as "polyA⁺ RNA".

In the context of the current invention, it is important that all or substantially all of the introns are retained in the pre-mRNA, i.e. they have not all been spliced out. As used herein, in one embodiment, the term "substantially all of the introns are retained" is used to mean that at least 50%, 60%, 70%, 80%, 90%, 95% or 99% of the intronic nucleotide sequence (which would ordinarily be spliced out) is retained in the pre-mRNA. In another embodiment, the term "substantially all of the introns are retained" is used to mean that at least 50%, 60%, 70%, 80%, 90%, 95% or 99% of the total number of introns are retained in the pre-mRNA.

In the context of the current invention, it is also important (but not essential) that polyadenylation has not occurred. This allows information to be obtained from the 3' end of the target gene. Therefore, the pre-mRNA may contain insubstantial or trace amounts of polyadenylated mRNA.

In some embodiments of the current invention, it is also important that the sequence variation around each allele is known. This allows sequence variation that affects gene regulation to be linked to allelic skew and gene dysregulation.

In the methods of the invention, the optimum amounts of pre-mRNA (i.e. as much as possible) are obtained from target eukaryotic cells.

Pre-mRNA may be obtained from cells by cell lysis followed by solvent extraction and depletion of rRNA and polyA⁺ RNA species. Pre-mRNA may also be obtained from cells by cell lysis followed by binding of RNA to an affinity column and depletion of rRNA and polyA⁺ RNA species.

Preferably, the pre-mRNA is obtained by cell lysis followed by solvent extraction and precipitation of total RNA. For example, total RNA can be extracted using TRIzol reagent (Sigma), PhaseLock Gel tubes (5Prime) and centrifugation. RNA can be precipitated by mixing with equal volumes of propan-2-ol followed by centrifugation. Precipitated RNA can be washed using 75% ethanol, dried and dissolved in water (e.g. Kowalczyk, 2012).

The pre-mRNA which is used in the methods of the invention may also be admixed with insubstantial amounts of other RNA, e.g. rRNA and/or tRNA, or DNA.

Preferably, the pre-mRNA does not comprise ribosomal RNA (rRNA) or the pre-mRNA has been depleted for rRNA, e.g. rRNA is removed from the pre-mRNA before use. For example, rRNA may be removed using the RiboMinus Eukaryote Kit for RNA sequencing (Invitrogen).

Preferably, the pre-mRNA is polyA⁻ mRNA. Preferably, the pre-mRNA does not comprise DNA; this may be removed with a DNAse.

Step (b) of the methods of the invention comprises determining the ratios (Rᵢⱼ) between the amounts of pre-mRNAs of one or more pairs of alleles (i,j) of the (same) genes.

In this regard, the methods of the invention encompass both (1) methods which involve the determination of the absolute amounts of pre-mRNA of pairs of alleles (and the ratio thereof) and (2) methods which determine the relative amounts of the pre-mRNA of pairs of alleles (without necessarily determining the absolute amounts of the pre-mRNA of the alleles).

Methods of obtaining the absolute amounts of pre-mRNA of alleles include RNA-Seq followed by genome-alignment and counting of the number of aligned sequences. Sequences deriving from specific alleles are identified and counted by identifying, within the RNA-Seq data, sequence changes in the introns, exons and downstream transcribed regions, known to be specific to that allele.

Preferably, the absolute amounts of pre-mRNA of the first and second alleles is determined by strand-specific RNA-Seq followed by genome-alignment and counting of the number of aligned sequences. Sequences that derive from the non-transcribed strand are discarded, to remove genomic contamination and antisense transcription. Sequences deriving from specific alleles are identified and counted by identifying, within the RNA-Seq data, sequence changes in the introns, exons and downstream transcribed regions, known to be specific to that allele (e.g. Quinn *et al.,* 2013).

Preferably, the relative or absolute amounts of pre-mRNA of the first and second alleles is determined using RNA-Seq.

Relative differences in the amounts of first and second alleles can be determined using hybridisation of cDNA to SNP microarrays and determining the relative signal from each allele. Relative differences in the amounts of first and second alleles can also be determined using SNP-specific PCR and determining the relative signal from each allele.

R_{i,j} is defined herein as the ratio of the amount of pre-mRNA of a pair of alleles (i,j) of the gene, wherein i is the first allele and j is the second allele.

In normal expression, the amount of pre-mRNA of the first allele (i) of the gene should be approximately equal to the amount of pre-mRNA of the second allele (j) of the gene, i.e. both alleles should be expressed equally.

When R_{i,j} ≠ 1 for one or more pairs of alleles (i,j) of the same gene, then this is indicative of dysregulation between the expression levels of those two alleles of that gene in that target eukaryotic cell.

In one embodiment, the term "R_{i,j} ≠ 1" means that Rᵢⱼ is substantially not equal to 1, e.g. Rᵢⱼ does not equal 1 when allowance is taken for random variation and experimental error.

In another embodiment, the term "R_{i,j} ≠ 1" means that the amount of pre-mRNA of the first allele (i) of the gene is statistically different from the amount of pre-mRNA of the second allele (j) of the gene (e.g. p<0.05 using Student t test).

Preferably, the term "R_{i,j} ≠ 1" means that R_{i,j} is less than 0.95, 0.9, 0.8, 0.7, 0.6, 0.5, 0.4, 0.3, 0.2, 0.1, 0.05 or 0.01; or R_{i,j} is more than 1.05, 1.1, 1.2, 1.4, 1.6, 2, 2.5, 3, 5, 10, 20 or 100. More preferably, R_{i,j} is less than 0.9 or R_{i,j} is more than 1.1.

For example, if R_{i,j} is < 0.9, then this is indicative of dysregulation between the expression of the first and second alleles of the gene. In this case, the level of expression of the first allele of the gene is less than the level of expression of the second allele of the gene.

For example, if R_{i,j} > 1.1, then this is indicative of dysregulation between the expression of the first and second alleles of the gene. In this case, the level of expression of the first allele of the gene is greater than the level of expression of the second allele of the gene.

The quantification of Rᵢⱼ between an affected allele (i) and a normal allele (j) allows for the reproducible and statistically-robust identification of genes linked to human diseases or traits. The degree of the change in Rᵢⱼ gives the direction of the genetics, i.e. showing whether an increase or decrease in the gene activity is associated with the disease or trait.

Furthermore, the size of Rᵢⱼ shows to what extent the gene activity needs to be change to have a measurable physiological effect.

If dysregulation is found between the pairs of alleles (i,j) of the gene, then the sequences of the pairs of alleles may be determined in order to try to establish the reason for the dysregulation, e.g. using RNA-Seq.

According to the invention, when R_{i,j} ≠ 1 for a pair of alleles (i,j) of a gene, or in response to determining that R_{i,j} ≠ 1 for a pair of alleles (i,j) of a gene, the method additionally comprises the steps of determining the nucleotide sequences of that pair of alleles.

RNA-Seq (RNA sequencing), also called whole transcriptome shotgun sequencing (WTSS), uses next-generation sequencing (NGS) to reveal the presence and quantity of RNA in a biological sample at a given moment.

In the context of the current invention, RNA-Seq comprises the following steps:
(i) The pre-mRNA is fragmented *in vitro* and copied into ds-cDNA (e.g. using reverse-transcriptase); and
(ii) The ds-cDNA is then sequenced, preferably using high-throughput, short-read sequencing methods (e.g. NGS).

These sequences may then be aligned to a reference genome sequence to reconstruct which genome regions were being transcribed. This data can be used to annotate where expressed genes are, their relative expression levels, and any alternative splice variants.

If the expression levels of the two alleles are different (e.g. R_{i,j} <0.9 or R_{i,j} >1.1), then this provides an indication that there exists a change in the regulatory elements on one allele that controls the expression of the gene. Regulatory elements can exist within the introns of the gene or outside of the body of the gene.

Methods such as ATAC-seq and NG Capture-C may also be used to further identify the precise genetic cause of the allelic dysregulation, particularly in cases where the cause is due to a change in regulatory elements.

The method of the invention may also comprise the step of carrying out a sequence-based assay (preferably ATAC-seq, DNase-seq or ChlP-seq) that measures the activity of regulatory elements to detect skew on the same allele of the genes found to be skewed in RNA-seq. The method of the invention includes using haplotype phasing.

### BRIEF DESCRIPTION OF THE FIGURES

Figure 1 shows the use of pre-mRNA in identifying gene dysregulation using phased haplotypes and pre-mRNA.
Figure 2 shows the use of pre-mRNA in phased genomes to detect dysregulation of the *IKZF1* gene associated with a specific sequence variant in a regulatory element.
Figure 3 shows the frequency with which informative heterozygotes are found in the general population for risk alleles associated with common disease.

### EXAMPLES

The present invention is further illustrated by the following Examples, in which parts and percentages are by weight and degrees are Celsius, unless otherwise stated. It should be understood that these Examples, while indicating preferred embodiments of the invention, are given by way of illustration only.

### Example 1: Use of pre-mRNA in phased genomes to detect gene dysregulation associated with a specific haplotype

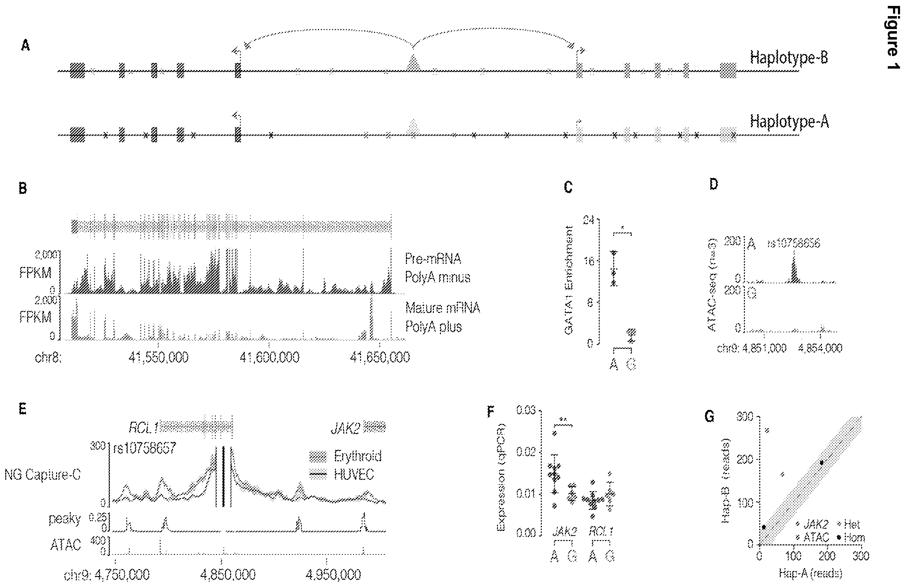

Figure 1A shows a schematic representation of two genome alleles each of which contain two genes and one regulatory element. Sequence changes which distinguish the two alleles are shown as Xs (for example, single nucleotide polymorphisms (SNPs), small insertions or deletions). The exons of the two genes are shown as boxes and the promoter elements of the two genes are shown as a vertical line and horizontal arrow associated with the first exon of the genes. The position of the regulatory element is shown as a triangle between the two genes.

In Haplotype A, this regulatory element contains a sequence change which alters its activity (shown as a lighter shade). The regulatory interactions between the regulatory element and genes, mapped by 3C methods such as Capture-C, are shown as arced lines with arrows. The sequence changes which distinguish the source allele of the pre-mRNAs from both genes lie within the transcribed portions of the genes (for example introns, exons and downstream regions). In this example the gene dysregulation is caused by a damaged regulatory element, but the same use of phased sequence changes combined with the sequencing of pre-mRNA can be used for any other mechanism (for example, gain-of-function caused by sequence variation or larger scale structural variation).

Figure 1B shows, in an exemplar gene, the increased coverage in pre-mRNA forms of RNA-Seq which retain the transcribed intronic and downstream regions of the gene and which increases the amount of sequence variation capable of detecting genes dysregulation. The exons of the gene are shown as vertical lines of varying thickness while the introns are shown as a horizontal hatched line.

Figure 1C shows the loss of binding of the GATA1 transcription factor (ChIP qPCR) at an erythroid regulatory element containing a single base pair change (rs10758656), homozygously edited into Hudep2 cells.

Figure 1D shows the loss of open chromatin signal at the same regulatory element using ATAC-seq in the homozygous presence of the sequence change rs10758656.

Figure 1E shows the erythroid-specific interaction between this regulatory element in wild type cells with the promoter of the *RCL1* and *JAK2.*

Figure 1F shows the loss of expression of only the *JAK2* gene in cells homozygously edited for rs10758656.

Figure 1G shows the allelic skew in primary erythroid cells toward the wild type allele for both ATAC-seq at the regulatory element (squares) and in pre-mRNA expression (circles) of the *JAK2* gene only in individuals heterozygous for rs10758656.

### Example 2: Use of pre-mRNA in phased genomes to detect dysregulation of the IKZF1 gene associated with a specific sequence variant in a regulatory element

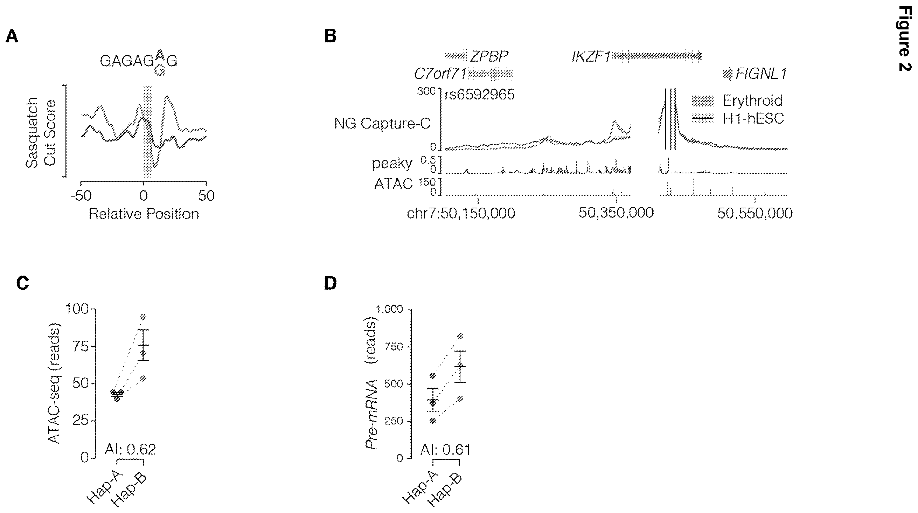

Figure 2A shows the identification of a sequence change in a regulatory element which damages the binding potential of a transcription factor using the Sasquatch algorithm (Schwessinger R. *et al.,* 2017).

Figure 2B shows that this regulatory element interacts erythroid-specifically with the promoter of the *IKZF1* gene using NG Capture-C.

Figure 2C shows the allelic skew of open chromatin signal towards the wild type (Hap-B) in primary erythroid cells in 3 individuals heterozygous for the damaging sequence change (Hap-A) as determined by ATAC-seq. The corresponding signal within the same sample in haplotype B is linked to the signal in haplotype A with a dotted line.

Figure 2D shows the cumulative decrease in pre-mRNA of the *IKZF1* gene haplotype A, summed up from all transcribed sequence changes which distinguish the two haplotypes. The corresponding signal within the same sample in haplotype B is linked to the signal in haplotype A with a dotted line.

### Example 3: The use of allelic skew in pre-mRNA in phased genomes allows for regulatory variation to be analysed at unprecedented scale in primary cells

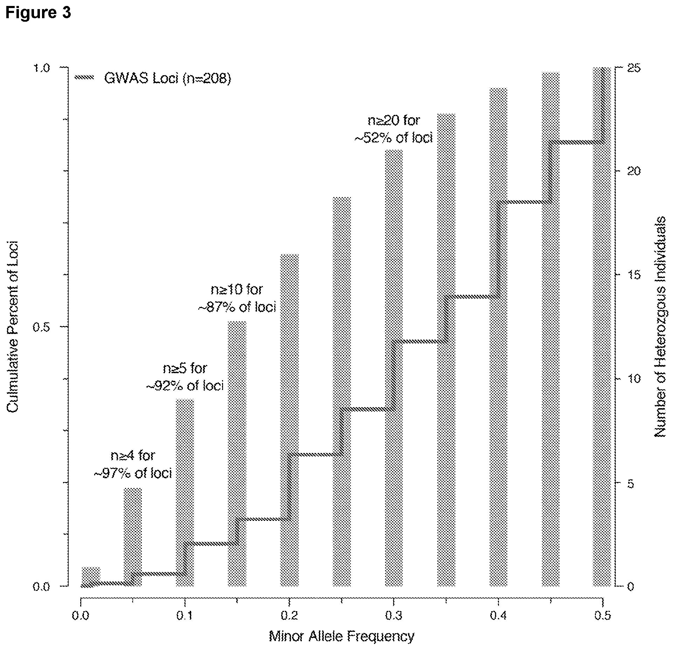

Figure 3 show the number of informative individuals expected for a given minor allele frequency (MAF) of the sequence variant in a random sampling of the general population. Grey bars represent the number of expected individuals that are heterozygous at a given MAF. The black line shows the average distribution of minor allele frequencies in a typical genome-wide association (GWA) study for human disease (Type 1 diabetes, ankylosing spondylitis, erythroid traits and multiple sclerosis, combined). This shows that, at a MAF of 0.3, this would provide greater than 20 independent observations of gene dysregulation and would cover greater than half of a typical GWA study. Similarly, for a MAF of 0.1, this would provide 5 or more independent observations of gene dysregulation and would cover greater than 90% of a typical GWA study.

### REFERENCES

James C et al., Cell, vol. 155, 2013, , "Human SNP Links Differential Outcomes in Inflammatory and Infectious Disease to a FOX03-Regulated Pathway", pages 57-69.
Kowalczyk, M.S. et al. Intragenic enhancers act as alternative promoters. Mol Cell 45, 447-58 (2012).
Quinn EM, et al. (2013) Development of Strategies for SNP Detection in RNA-Seq Data: Application to Lymphoblastoid Cell Lines and Evaluation Using 1000 Genomes Data. PLoS ONE 8(3): e58815. https://doi.org/10.1371/journal.pone.0058815 .
Rainbow et al., BIOCHEMICAL SOCIETY TRANSACTIONS, vol. 36, 2008, "Commonality in the genetic control of Type 1 diabetes in humans and NOD mice: variants of genes in the IL-2 pathway are associated with autoimmune diabetes in both species", page 312 .
Schwessinger R, et al. (2017) Sasquatch: predicting the impact of regulatory SNPs on transcription factor binding from cell- and tissue-specific DNase footprints. Genome Res. 2017 Oct;27(10):1730-1742. PMCID: PMC5630036.
Sigurdsson et al., HUMAN MOLECULAR GENETICS, vol. 17, 2008, "A risk haplotype of STAT4 for systemic lupus erythematosus is over-expressed, correlates with anti-dsDNA and shows additive effects with two risk alleles of IRF5", pages 2868-2876 .
Thomas et al., EPIGENETICS & CHROMATIC, vol. 4, 2011, "Allele-specific transcriptional elongation regulates monoallelic expression of thelGF2BPI gene", page 14.

## Claims

1. A method of identifying mutations in alleles of a gene which may be causative of dysregulation of the expression levels of the alleles of the gene in a target eukaryotic cell,
the method comprising the steps of:
for a plurality of genes from one or more target eukaryotic cells,
(a) obtaining pre-mRNAs of at least two alleles of the genes; and
(b) determining the ratios (R_{i,j}) between the amounts of pre-mRNAs of one or more pairs of alleles (i,j) of the genes;
wherein when R_{i,j} ≠ 1 for a pair of alleles (i,j) of a gene,
or in response to determining that R_{i,j} ≠ 1 for a pair of alleles (i,j) of a gene,
the method additionally comprises the steps:
(c) determining the nucleotide sequences of that pair of alleles; and
(d) comparing the nucleotide sequences of that pair of alleles in order to identify sequence differences between the nucleotide sequences of that pair of alleles;
(e) attributing all of the sequence differences to a specific allele to determine allelic skew across the whole gene;
wherein the method is performed in a phased genome sequence, and
(f) linking these sequence differences with sequence variation outside the body of the gene.

2. A method as claimed in claim 1, wherein Step (c) is carried out using RNA-Seq.

3. A method as claimed in claim 2, wherein sequences deriving from specific alleles are identified and counted by identifying, within the RNA-Seq data, sequence changes in the introns, exons and downstream transcribed regions, known to be specific to that allele.

4. A method as claimed in any one of the preceding claims, wherein the eukaryotic cells are human primary lymphoid cells or primary neuronal cells.

5. A method as claimed in any one of the preceding claims, wherein the plurality of genes is 2-10, 10-100, 100-500, 500-1000, 1000-5000, 5000-10000, or 10000 or more genes.

6. A method as claimed in any one of the preceding claims, wherein there are 2 alleles of the same gene in each target eukaryotic cell.

7. A method as claimed in any one of the preceding claims, wherein the pre-mRNA is polyA⁻ mRNA.

8. A method as claimed in claim 7, wherein the pre-mRNA is polyA- mRNA obtained from total cellular RNA.

9. A method as claimed in any one of the preceding claims, wherein R_{i,j} ≠ 1 means that R_{i,j} is less than 0.95, 0.9, 0.8, 0.7, 0.6, 0.5, 0.4, 0.3, 0.2, 0.1, 0.05 or 0.01; or R_{i,j} is more than 1.05, 1.1, 1.2, 1.4, 1.6, 2, 2.5, 3, 5, 10, 20 or 100.

10. A method as claimed in any one of the preceding claims, wherein the method further comprises detecting aberrant expression of genes which may be associated with a disease or disorder using haplotype phasing.

11. A method as claimed in any one of the preceding claims, wherein the method further comprises linking the skew in gene expression to genetic signals associated with traits and disease traits outside the body of the gene, where the traits are enriched.

12. A method as claimed in any one of the preceding claims, wherein the method further comprises:
(a) linking of the skew in gene expression to skew in sequence-based genomics assays linked to regulatory activities, to validate the mechanism underlying the changes in gene expression; or
(b) linking of the skew in gene expression to skew in sequence-based genomics assays linked to regulatory activities *en masse* and at genome-scale, to validate the mechanism underlying the changes in gene expression.

13. A method as claimed in any one of the preceding claims, wherein the sequence variation outside the body of the gene causes gene dysregulation on the affected allele.

14. A method as claimed in any one of the preceding claims, wherein the method further comprises:
(a) using information from the method to determine the underlying genetic cause of a disease or disorder; or
(b) using information from the method to identify genes that are aberrantly expressed in specific diseases or disorders (or their associated biochemical pathways), and thereby identifying candidate drug targets.

15. A method as claimed in any one of the preceding claims, wherein one or more of the differences between the nucleotide sequences of the pair of alleles of the gene may be mutations which are causative of the dysregulation of the expression levels of the two alleles of that gene in the target eukaryotic cell.

## Patentansprüche

1. Ein Verfahren zur Identifikation von Mutationen in Allelen eines Gens, das eine Regulationsstörung der Expressionslevels der Allele des Gens in einer Eukaryoten-Zielzelle verursachen kann,
wobei das Verfahren die folgenden Schritte umfasst:
für eine Vielzahl von Genen von einer oder mehreren Eukaryoten-Zielzellen,
(a) Erhalten von Prä-mRNAs von mindestens zwei Allelen der Gene; und
(b) Bestimmen der Verhältnisse (R_{i,j}) zwischen den Mengen der Prä-mRNAs des einen oder der mehreren Paare von Allelen (i,j) der Gene;
wobei, wenn R_{i,j} ≠ 1 für ein Paar von Allelen (i,j) eines Gens ist,
oder als Antwort auf das Bestimmen, dass R_{i,j} ≠ 1 für ein Paar von Allelen (i,j) eines Gens,
das Verfahren zusätzlich die folgenden Schritte umfasst:
(c) Bestimmen der Nukleotidsequenzen dieses Paars von Allelen; und
(d) Vergleichen der Nukleotidsequenzen dieses Paars von Allelen, um Sequenzunterschiede zwischen den Nukleotidsequenzen dieses Paars von Allelen zu identifizieren;
(e) Zuweisen aller Sequenzunterschiede zu einem spezifischen Allel, um den Allelversatz über das gesamte Gen hinweg zu bestimmen;
wobei das Verfahren in einer phasierten Genomsequenz durchgeführt wird, und
(f) Verknüpfen dieser Sequenzunterschiede mit Sequenzvariation außerhalb des Körpers des Gens.

2. Verfahren nach Anspruch 1, wobei Schritt (c) unter Verwendung von RNA-Seq ausgeführt wird.

3. Verfahren nach Anspruch 2, wobei aus spezifischen Allelen abgeleitete Sequenzen identifiziert und gezählt werden, indem innerhalb der RNA-Seq-Daten Sequenzänderungen in den Introns, Exons und stromabwärts transkribierten Regionen, von denen bekannt ist, dass sie spezifisch für dieses Allel sind, identifiziert werden.

4. Verfahren nach einem der vorhergehenden Ansprüche, wobei die Eukaryotenzellen menschliche primäre Lymphoidzellen oder primäre neuronale Zellen sind.

5. Verfahren nach einem der vorhergehenden Ansprüche, wobei die Vielzahl von Genen 2-10, 10-100, 100-500, 500-1000, 1000-5000, 5000-10000 oder 10000 oder mehr Gene ist.

6. Verfahren nach einem der vorhergehenden Ansprüche, wobei es 2 Allele des gleichen Gens in jeder Eukaryoten-Zielzelle gibt.

7. Verfahren nach einem der vorhergehenden Ansprüche, wobei die Prä-mRNA PolyA⁻-mRNA ist.

8. Verfahren nach Anspruch 7, wobei die Prä-mRNA PolyA⁻-mRNA ist, die aus gesamter zellulärer RNA erhalten wurde.

9. Verfahren nach einem der vorhergehenden Ansprüche, wobei R_{i,j} ≠ 1 bedeutet, dass R_{i,j} weniger als 0,95, 0,9, 0,8, 0,7, 0,6, 0,5, 0,4, 0,3, 0,2, 0,1, 0,05 oder 0,01 ist; oder dass R_{i,j} mehr als 1,05, 1,1, 1,2, 1,4, 1,6, 2, 2,5, 3, 5, 10, 20 oder 100 ist.

10. Verfahren nach einem der vorhergehenden Ansprüche, wobei das Verfahren ferner das Detektieren einer aberrierenden Expression von Genen, die mit einer Erkrankung oder Störung assoziiert sein können, unter Verwendung von Haplotyp-Phasierung umfasst.

11. Verfahren nach einem der vorhergehenden Ansprüche, wobei das Verfahren ferner das Verknüpfen des Versatzes in der Genexpression mit genetischen Signalen, die mit Merkmalen und Erkrankungsmerkmalen außerhalb des Körpers des Gens assoziiert sind, wo die Merkmale angereichert sind, umfasst.

12. Verfahren nach einem der vorhergehenden Ansprüche, wobei das Verfahren ferner Folgendes umfasst:
(a) Verknüpfen des Versatzes in der Genexpression mit dem Versatz in sequenzbasierten Genomik-Assays, die mit regulatorischen Aktivitäten verknüpft sind, um den Mechanismus, der den Genexpressionsänderungen zugrunde liegt, zu validieren; oder
(b) Verknüpfen des Versatzes in der Genexpression mit dem Versatz in sequenzbasierten Genomik-Assays, die mit regulatorischen Aktivitäten verknüpft sind, in der Masse und im Genom-Maßstab, um den Mechanismus, der den Genexpressionsänderungen zugrunde liegt, zu validieren.

13. Verfahren nach einem der vorhergehenden Ansprüche, wobei die Sequenzvariation außerhalb des Körpers des Gens eine Regulierungsstörung an dem betroffenen Allel verursacht.

14. Verfahren nach einem der vorhergehenden Ansprüche, wobei das Verfahren ferner Folgendes umfasst:
(a) Verwenden von Informationen aus dem Verfahren, um die zugrunde liegende genetische Ursache einer Erkrankung oder Störung zu bestimmen; oder
(b) Verwenden von Informationen aus dem Verfahren, um Gene zu identifizieren, die aberrierend in spezifischen Erkrankungen oder Störungen (oder den mit ihnen assoziierten biochemischen Stoffwechselwegen) exprimiert werden, und somit Identifizieren von Arzneimittelziel-Kandidaten.

15. Verfahren nach einem der vorhergehenden Ansprüche, wobei einer oder mehrere der Unterschiede zwischen den Nukleotidsequenzen des Paars von Allelen Mutationen sein können, die die Regulationsstörung der Expressionslevels der zwei Allele dieses Gens in der Eukaryoten-Zielzelle verursachen.

## Revendications

1. Procédé d'identification de mutations dans des allèles d'un gène pouvant être à l'origine d'une dérégulation des niveaux d'expression des allèles du gène dans une cellule eucaryote cible,
le procédé comprenant les étapes qui consistent à :
pour une pluralité de gènes issus d'une ou plusieurs cellules eucaryotes cibles,
(a) obtenir des pré-ARNm d'au moins deux allèles des gènes ; et
(b) déterminer les ratios (R_{i,j}) entre les quantités de pré-ARNm d'une ou plusieurs paires d'allèles (i,j) des gènes ;
dans lequel lorsque Ri,j ≠ 1 pour une paire d'allèles (i,j) d'un gène,
ou en réponse à la détermination que Ri,j ≠ 1 pour une paire d'allèles (i,j) d'un gène, le procédé comprend en outre les étapes :
(c) de détermination des séquences nucléotidiques de cette paire d'allèles ; et
(d) de comparaison des séquences nucléotidiques de cette paire d'allèles afin d'identifier des différences de séquence entre les séquences nucléotidiques de cette paire d'allèles ;
(e) d'attribution de toutes les différences de séquence à un allèle spécifique pour déterminer un biais allélique sur l'ensemble du gène ;
le procédé étant mis en œuvre dans une séquence de génome phasé, et
(f) d'établissement d'un lien entre ces différences de séquence et la variation de séquence en dehors du corps du gène.

2. Procédé selon la revendication 1, dans lequel l'étape (c) est réalisée en utilisant ARN-Seq.

3. Procédé selon la revendication 2, dans lequel des séquences dérivées d'allèles spécifiques sont identifiées et comptées en identifiant, dans les données d'ARN-Seq, des changements de séquence dans des introns, des exons et des régions transcrites en aval, connus pour être spécifiques à cet allèle.

4. Procédé selon l'une quelconque des revendications précédentes, dans lequel les cellules eucaryotes sont des cellules lymphoïdes primaires humaines ou des cellules neuronales primaires.

5. Procédé selon l'une quelconque des revendications précédentes, dans lequel la pluralité de gènes est de 2-10, 10-100, 100-500, 500-1 000, 1 000-5 000, 5 000-10 000 ou 10 000 gènes ou plus.

6. Procédé selon l'une quelconque des revendications précédentes, dans lequel il y a 2 allèles du même gène dans chaque cellule eucaryote cible.

7. Procédé selon l'une quelconque des revendications précédentes, dans lequel le pré-ARNm est un ARNm polyA-.

8. Procédé selon la revendication 7, dans lequel le pré-ARNm est un ARNm polyA- obtenu à partir d'ARN cellulaire total.

9. Procédé selon l'une quelconque des revendications précédentes, dans lequel Ri,j ≠ 1 signifie que R_{i,j} est inférieur à 0,95, 0,9, 0,8, 0,7, 0,6, 0,5, 0,4, 0,3, 0,2, 0,1, 0,05 ou 0,01 ; ou R_{i,j} est supérieur à 1,05, 1,1, 1,2, 1,4, 1,6, 2, 2, 5, 3, 5, 10, 20 ou 100.

10. Procédé selon l'une quelconque des revendications précédentes, le procédé comprenant en outre la détection, par phasage des haplotypes, d'une expression aberrante de gènes pouvant être associée à une maladie ou une affection.

11. Procédé selon l'une quelconque des revendications précédentes, le procédé comprenant en outre l'établissement d'un lien entre le biais dans l'expression génique et des signaux génétiques associés à des traits et à des traits pathologiques en dehors du corps du gène, où les traits sont enrichis.

12. Procédé selon l'une quelconque des revendications précédentes, le procédé comprenant en outre :
(a) l'établissement d'un lien entre le biais dans l'expression génique et le biais dans des tests génomiques basés sur la séquence correspondant à des activités régulatrices, pour valider le mécanisme sous-jacent aux changements dans l'expression génique ; ou
(b) l'établissement d'un lien entre le biais dans l'expression génique et le biais dans des tests génomiques basés sur la séquence correspondant à des activités régulatrices, réalisés en masse et à l'échelle du génome, pour valider le mécanisme sous-jacent aux changements dans l'expression génique.

13. Procédé selon l'une quelconque des revendications précédentes, dans lequel la variation de séquence en dehors du corps du gène provoque une dysrégulation génique sur l'allèle concerné.

14. Procédé selon l'une quelconque des revendications précédentes, le procédé comprenant en outre :
(a) l'utilisation d'informations issues du procédé pour déterminer la cause génétique sous-jacente d'une maladie ou d'une affection ; ou
(b) l'utilisation d'informations issues du procédé pour identifier des gènes qui sont exprimés de manière aberrante dans des maladies ou des affections spécifiques (ou les voies biochimiques qui leur sont associées), et identifier ainsi des cibles potentielles pour un médicament.

15. Procédé selon l'une quelconque des revendications précédentes, dans lequel une ou plusieurs des différences entre les séquences nucléotidiques de la paire d'allèles du gène peuvent être des mutations responsables de la dysrégulation des niveaux d'expression des deux allèles de ce gène dans la cellule eucaryote cible.
